(19) Europäisches Patentamt
European Patent Office
Office européen des brevets

(11) EP 2 172 473 A1

(12) **EUROPEAN PATENT APPLICATION**

(43) Date of publication:
**07.04.2010 Bulletin 2010/14**

(21) Application number: **09002251.8**

(22) Date of filing: **21.08.2003**

(51) Int Cl.:
***C07H 7/06*** (2006.01) ***C07H 9/04*** (2006.01)
***C07D 309/32*** (2006.01) ***C07H 3/10*** (2006.01)
***A01N 43/08*** (2006.01) ***A01N 43/16*** (2006.01)
***A01N 43/90*** (2006.01)

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **03.04.2003 PT 10293503**

(62) Document number(s) of the earlier application(s) in accordance with Art. 76 EPC:
**03398005.3 / 1 464 649**

(71) Applicants:
- **Instituto Politécnico de Santarém / Escola Superior Agrária 2001-904 Santarém (PT)**
- **Faculdade De Ciências da Universidade de Lisboa 1749-016 Lisboa (PT)**
- **University of Newcastle Newcastle upon Tyne NE1 7RU (GB)**

(72) Inventors:
- **Guerra Justino, Jorge, Alberto 2000-019 Santarém (PT)**
- **Pilar Grases Santos Silva Reuter, Amélia 1000-239 Lisboa (PT)**
- **Lukeba Canda, Tana Luanda (AO)**
- **Wilkins, Richard Newcastle-upon-Tyne NE2 2DR (GB)**

(74) Representative: **Pereira da Cruz, Joao J. Pereira da Cruz, S.A. Rua Vitor Cordon, 14 1249-103 Lisboa (PT)**

Remarks:
This application was filed on 18-02-2009 as a divisional application to the application mentioned under INID code 62.

(54) **Alpha, beta-unsaturated, delta-lactones, process for their preparation and their application as pesticides**

(57) The present invention relates to compounds of general formula (1):

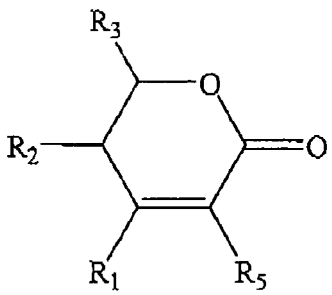

(I)

wherein $R_1$ and $R_2$ represent, independently from each other, hydrogen, halogen, alkoxy, substituted alkoxy or an ester group; $R_3$ represents $-CH_2R_6$ (wherein $R_6$ represents an ester group), oxiranyl, or a group of formula

EP 2 172 473 A1

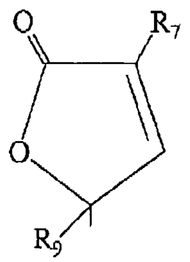

or

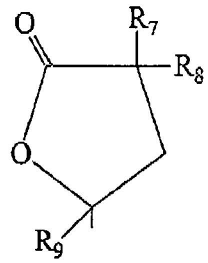

wherein $R_7$ represents hydrogen or alkyl; $R_9$ represents phenyleulfanyl, phenylselenyl, phenylsulfoxy or phenyl-selenoxy; and $R_9$ represents hydrogen or halogen; as well as to the process for the preparation of these compounds, their utilization as pesticides and to the method to apply the same to the said pests or their locus.

## Description

### Field of the Invention

The present invention relates α,ß-unsaturated δ-lactones with pesticidal activity, particularly insecticidal, to processes for their preparation and to their utilization as pesticides, which are particularly effective against fruit fly, housefly and whitefly.

### Background of the Invention

Compounds with the α,β-unsaturated γ-lactone moiety in their structure occur in the plant kingdom, as metabolites of lichens and fungi,[1] as sesquiterpene derivatives[2] or as steroid glycosides derivatives.[3]

Natural products possessing this structural element are also components of animal species such as sponges.[4]

Many of these compounds exhibit a variety of biologic activities such as antifungal, insecticidal, antibacterial, phytotoxic and anti-inflammatory. Some of them are antibiotics, potential anticancer agents and cyclooxygenase or phospholipase $A_2$ inhibitors.[5]

Due to their biological importance, several synthetic methods have been developed for the preparation of α, β-unsaturated γ-lactones. The synthesis of the endocyclic lactones (α,β-butenolides) is reported in the literature, and includes mercuration-carbonylation of propargylic alcohols,[6] condensation of 2,5-bis(trimethylsiloxy)furans with carbonyl compounds in the presence of titanium tetrachloride[7] and various transformations of $C_3$ synthons, such as, for example, glycidaldehyde.[8]

Other references report methods leading to the synthesis of γ-alkylidene-α,β-butenolides[9] and to the preparation of α,β-butenolide derivatives with insect antifeedant activity.[10]

A method leading to the preparation of the exocyclic type lactones involves the reaction of 2-(bromomethyl) acrylic acid in the presence of indium with carbonyl compounds, to give α-methylene-y-butyrolactones in 7-96% yield.[11]

Previous work reports the synthesis of butenolides through the condensation of sugar epoxides with the dianion of phenylselenoacetic acid, followed by hydrolysis and subsequent oxidation of the intermediate phenylselenolactone.[12,13,14] The nucleophilic opening of the oxirane is stereospecific, the configuration of the stereogenic centre in the final lactone being determined by the centre of chirality of the starting epoxide.

Another method for the synthesis of α,β-unsaturated γ-lactones involves a Reformatsky type reaction of a ketosugar or a dialdofuranose with ethyl bromomethylacrylate and zinc in THF under reflux.[13,14,15]

Ethyl bromomethylacrylate and zinc-silver/graphite at -78°C have been successfully applied to the synthesis of hydroxyesters from cyclic ketones,[16] ketosugars and a 2,3-*O*-isopropylidene-D-erythronolactone[17] and to the synthesis of α,β-unsaturated γ-lactones from some ketosugars.[16]

The synthesis of 3-ulosonic acids via a samarium iodide Reformatsky reaction of aldonolactones was also reported.[18]

Some of this type of compounds, reported in the literature, have fungicidal efficacy.[13]

Epoxy sugars are versatile intermediates in organic synthesis, due to the ease of their preparation from a variety of starting compounds and due to their susceptibility to reactions for example with electrophiles, nucleophiles, acids and bases. Furthermore, epoxides are part of the range of compounds recognized as active principles, with biological and pharmacological activity.[19] Reference can be made for example to cytotoxic metabolites, namely crotepoxide, pipoxide and senepoxide, the latter playing an important role in plants as an antiparasitic.[20]

Methods for the preparation of epoxysugars use halohydrins as intermediate compounds,[21] and also aminosugars,[22] tosylates and/or mesylates,[23] vicinal diols,[24,25] glycals[26] and carbonyl compounds.[27]

### Summary of the Invention

This invention is related to the synthesis and pesticidal application of compounds of general formula (I) described further on.

These compounds possess efficacy as insecticides with high toxicity to fruit fly (*Drosophila melanogaster*), housefly (Musca *domestica*) and whitefly (*Trialeurodes vaporarium*).

On the contrary the compounds are not toxic to brine shrimps (*Artemia salina*), the reference organisms in assays to evaluate the potential toxicity hazard to organisms in ecosystems.

### Detailed Description of the Invention

A first object of the invention is a family of compounds with pesticidal activity, of general formula (I):

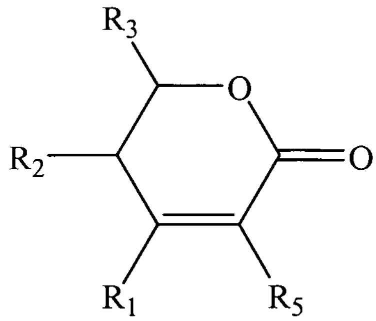

(I)

wherein

| | |
|---|---|
| $R_1$ and $R_2$ | represent, independently from each other, hydrogen, halogen, alkoxy, substituted alkoxy or an ester group; or |
| $R_3$ | represents $-CH_2R_6$, wherein $R_6$ represents an ester group,oxiranyl, or a group of formula |

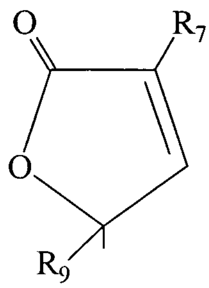

or

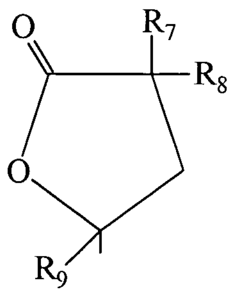

wherein
$R_7$ represents hydrogen or alkyl,
$R_8$ represents phenylsulfanyl, phenylselenyl, phenylsulfoxy or phenylselenoxy, and
$R_9$ represents hydrogen, ethoxycarbonyl or carbamoyl; and

| | |
|---|---|
| $R_5$ | represents hydrogen or halogen. |

Among this family of compounds of formula (I) are preferred those compounds of general formula (I'):

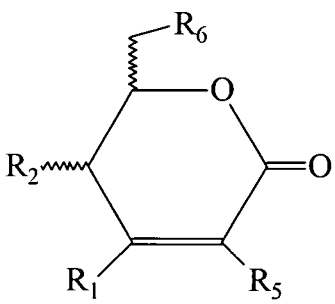

(I')

wherein $R_1$, $R_2$, $R_5$ and $R_6$ have the same meaning as defined for formula (I).

Especially preferred are those compounds of formula (I') wherein $R_1$ represents hydrogen, $R_2$ represents -OC(=O)$CH_3$, $R_5$ represents Br and $R_6$ represents -OC(=O)$CH_3$.

Among these, the most especially preferred is the D-*eritro* derivative, *i.e.*, 4,6-di-*O*-acetyl-2-bromo-2,3-dideoxy-D-eritro-hex-2-enono-1,5-lactone.

A second object of this invention is the process for the preparation of compounds of formula (I').

**Synthesis of compounds of Formula (I')**

Compounds of formula (I') can be prepared according to **Scheme 1**.

**Scheme 1**

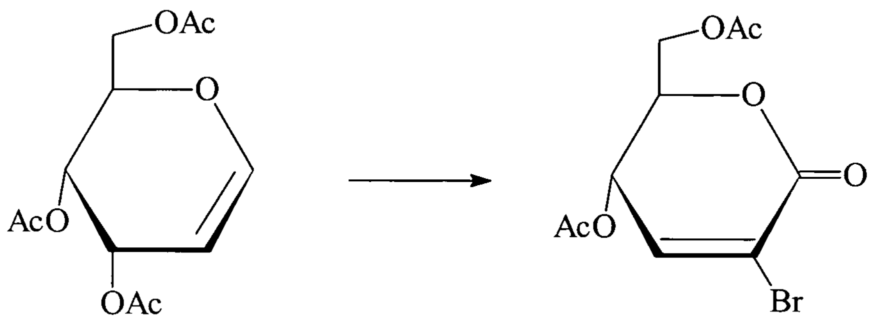

(II) (I'')

In this **Scheme 1**, a compound of formula (II) is reacted with *N*-bromosuccinimide in the presence of tetrahydrofuran and water, at a temperature of 10°C to 50°C, for a period of time from 4 to 24 hours. In a second step the obtained product is added to molecular sieves and pyridinium chlorochromate in dichloromethane, at a temperature of 10°C to 50°C, for a period of time from 8 to 24 hours, so that a α,β-unsaturated hexono-1,5-lactone of formula (I") [compound of formula (I'), wherein $R_1$ represents hydrogen, $R_2$ and $R_6$ represent acetoxy and $R_5$ represents bromo].

A third object of this invention is the use of compounds of formula (I) as pesticides.

Preferably, these compounds are used as arthropodicides.

More preferably, these compounds are used as insecticides.

Even more preferably, these compounds are used with special efficacy as insecticides with high toxicity in the control of fruit fly *(Drosophila melanogaster),* housefly (*Musca domestica*) and whitefly (*Trialeurodes vaporarium*).

A fourth object of the invention is a method to control pests, namely arthropods, particularly insects, especially the fruit fly, housefly and whitefly. The said method comprises the application of an effective amount of compounds of formula (I) to the above-mentioned pests or their locus.

**EXPERIMENTAL**

**Preparation Examples**

Example 1

Preparation of compound 4,6-di-*O*-acetyl-2-bromo-2,3-dideoxy-D-eritro-hex-2-enono-1,5-lactone **(1)**

*N*-bromosuccinimide (780 mg, 4.88 mmol) was added to a solution of tri-*O*-acetyl-D-glucal (1 g, 3.67 mmol) in 370 mL tetrahydrofuran (THF) and 92 mL distilled water. The mixture was stirred overnight at room temperature. The reaction mixture was poured into water chilled to 0 °C, followed by extraction of the mixture thus obtained with diethyl ether. The combined organic phases were dried over sodium sulphate and, after filtration, the solvent was removed in the rotatory evaporator and a syrupy residue was obtained. The residue was then added to a suspension of 3 Å (6.8 g) molecular sieves and pyridinium chloroformate (4.4 g) in dichloromethane (20 mL). The reaction mixture was stirred at

room temperature overnight, eluted with diethyl ether and stirred at room temperature for 10 min. The precipitate was removed by filtration and filtered under vacuum. The colourless filtrate was taken to the rotatory evaporator and the title compound was obtained as syrup (760 mg, $\eta$ = 37%). $[\alpha]_D^{20}$ = +121 (c 1, $CH_2Cl_2$); IR (neat): 1752 $cm^{-1}$ (C=O, lactone); 1632 $cm^{-1}$ (C=C) ; $^1H$ NMR (300 MHz, $\delta$, ppm, J=Hz, $CDCl_3$) : $\delta$ 7.01 (d, 1 H, H-3, $J_{3,4}$ = 3.9); 5.30 (dd, 1 H, H-4, $J_{4,5}$ = 6.6); 4.19, 4.17, 4.15, 4.13 (H-6a, part A of AB system, $J_{6a,6b}$ = 12.6, $J_{5,6a}$ = 4.5) ; 4.10, 4.08, 4.06, 4.04 (H-6b, part B of AB system, $J_{5,6b}$ = 3.6); 1.91 (s, 3 H, acetyl $CH_3$) ; 1.85 (s, 3 H, acetyl $CH_3$) ; $^{13}C$ NMR (75.43 MHz, $\delta$, ppm, $CDCl_3$): $\delta$ 170.1, 169.0 (acetyl C=O); 157.1 (lactone C=O); 142.3 (C-3); 116.4 (C-2); 77.7 (C-4); 64.8 (C-5); 61.6 (C-6), 20.4 (acetyl $CH_3$); Calculated elemental analysis for $C_{10}H_{11}O_6Br$ (307.08) : C 39.12; H 3.61; Experimental value: C 39.13; H 3.62.

**Biological Activity of the Compounds**

**Materials and Methods for Determination of Biological Activity**

A range of arthropod species was chosen to represent those in the terrestrial, aerial and aquatic environment, covering important target pest groups such as housefly, fruit fly and the whitefly, which belongs to a group of agricultural and horticultural pests.

Method A

Topical treatment of adult fruit flies *(D. melanogaster)*

A culture of fruit flies (*D. melanogaster*) was used in the production of adult flies (approx 0.22 mg) of about seven days.

Solutions of the compounds were prepared in acetone, and volumes of approximately 0.2 $\mu$L were applied to the ventral surface of the insects, using a calibrated PAX 100 microapplicator and a 1 mL syringe.

The flies, in groups of 5, were anaesthetized using carbon dioxide. Later on they were place in a bottle kept at 30+1° C, for observation of mortality at 1, 2, 3 and 24 hours after treatment.

For this purpose, solutions of 6 different concentrations were used in groups of 12 and 20 insects. A blank test was used as control. The mortality in the control test was normally zero but occasionally rose to 5-10.

Method B

Second method for topical treatment of adult fruit flies

Different dilutions of the compounds in acetone were prepared and individually applied to fruit flies using a Gilson piston micropipette.

Each fly was immobilized and 1 $\mu$L of acetone solution was applied. After evaporation of the acetone the fly was placed into a bottle, kept at 30$\pm$1° C, for observation following standard methodology.

Method C

Method by feeding the adult fruit fly *(D. melanogaster)*

Glass containers are employed, provided with capsules inside of which is inserted a piece of cotton wool. This was soaked in a 10% sugar solution containing the compounds to be tested in a determined concentration.

Care was taken to ensure that no solution dripped from the cotton wool and condensation was avoided by keeping the containers at room temperature (25° C).

The flies were anaesthetized using carbon dioxide and placed in the containers for recovery and feeding.

Method D

Topical treatment of the fruit fly larvae

Fruit fly larvae were separated from the growing medium and during the second and third instars were topically

treated with 0.2 μl of the acetone solutions of the compounds to be tested using a PAX microapplicator.

Larvae were immobilized for dosing and then placed on moistened filter paper, at 30±1°C, for observation.

Method E

Topical treatment of the adult housefly *(M. domestica)*

A Cooper-type culture was developed (to test behaviour in presence of insecticides). Three-day-old adult houseflies (body weight about 1.8 mg) were anaesthetized with carbon dioxide. Using a PAX microapplicator, a volume of 1 μl of the test compounds in acetone solution was applied to the dorsal cuticle of the fly, keeping it immobilized.

Groups of flies are placed into closed containers, kept at 30±1° C, for observation.

Method F

Immersion bioassays of *Artemia salina* shrimp larvae in brine

Freshly hatched shrimp larvae were obtained after the addition of shrimp eggs to a brine solution (15 g of salt in one litre of water). The next day larvae were separated from eggs and empty egg shells, using their phototropic movement and a Pasteur pipette.

Using a micropipette, 195 μL of brine containing 5 shrimp larvae, were dispensed into small glass containers. The solution of the compound to be tested in 5μ L acetone was then added, using 6 different concentrations. The container was closed and kept at 30±1° C for observation.

Dead and alive shrimps were counted at 1 and 24 hours after treatment, using a microscope.

A blank test was performed for comparison of the results.

Method G

Foliar treatment in glasshouses against the whitefly (*T. vaporariorum)*

Seedlings of tomato plants were infested with adult whiteflies. Selected leaves of the tomato plants were excised and carefully arranged in groups of 3. Care was taken for not disturbing the infestation. Each group was placed in a glass tube and, later on, the leaves were sprayed on both sides using, for each one, 200 μL of the solution of the compound to be tested in 30% acetone in water.

Controls were sprayed with the solvent alone.

Counts of insects were made on each leaf immediately after spraying and after 14 hours.

Calculation of toxicity parameters

Dosages used in insect treatments were based upon the amount of compound applied to each insect. In the case of shrimps, final concentrations of the compounds in the immersion brine medium were used.

Mortality after 24 hours was used to calculate the $LD_{50}/LC_{50}$ using linear regression analysis of the probability of mortality percentage as a function of the dose/concentration log.[30] This calculation was performed using PoloPC software (LeOra Software, Berkeley, CA, 1994).

There is no available statistics in respect of single dose treatments used (in the case of whitefly assays), and the results are expressed as effect percentage.

**Results**

Toxicity to the fruit fly (*D. Melanogaster)*

The results of assays with the fruit fly D. *Melanogaster* are shown in Table 1.

The table does also show confidence intervals at 95% for the $LD_{50}$ values, the number of tested organisms, the slope obtained by linear regression and the g index (of significance). The values are considered satisfactory if g is substantially lower than 1 and normally not higher than 0.4.[31]

As regards confidence intervals for $LD_{50}$, the indication (90%) means that intervals were calculated at 90% and not at 95%.

From the analysis of the $LD_{50}$ values, calculated by method 1, it is found that the compound tested in Example 1 is quite active against the adult fruit fly.

The activity of this compound is shown to be higher than that of "imidacloprid", the reference insecticide for the fruit fly.

Table 1 - Toxicity parameters of the compounds tested on fruit flies according to methods A and D.

| Compound No. | $LD_{50}$ (μg/ insect) | Confidence Intervals at 95% for $LD_{50}$ (μg/insecto) | No. of Organisms Tested | Slope | g |
|---|---|---|---|---|---|
| **Method A** | | | | | |
| 1 | 0.00016 | 0.00000 - 0.00226 | 114 | 0.296±0.096 | 0.401 |
| **Imidacloprid** | 0.01253 | 0.00523 - 0.01637 | 75 | 2.218±0.911 | 0.648 |
| | | | | | |
| **Method D** | | | | | |
| 1 | 4.74974 | 3.21963 - 20.59868 (90%) | 75 | 1.602±0.674 | 0.681 |
| n.d.- Non-available data | | | | | |

Fruit fly larvae are less sensitive to toxins than adult flies.

The $LD_{50}$ value obtained for the compound of Example 1 is much higher than that obtained for the adult fly. Therefore this compound is less toxic for larvae than for adult flies.

Toxicity to the housefly *M. domestica*

Toxicity parameters for adult houseflies, topically treated with the compounds of Example 1, are shown in Table 2.

Although these insects are approximately 8 fold larger than fruit flies, the analysis of the said table permits to conclude that the tested compound is much less toxic (2 to 3 orders of magnitude) for this type of flies than for fruit flies.

$LD_{50}$ values do only correspond to a moderate insecticidal activity.

Table 2 - Toxicity parameters of compounds tested on adult houseflies

| Compound No. | $LD_{50}$ (μg/ insect) | Confidence Intervals at 95% for $LC_{50}$ (μg/insect) | No. of Organisms Tested | Slope | g |
|---|---|---|---|---|---|
| **Method E** | | | | | |
| 1 | 0.06481 | n.d. | 40 | 10.228±0.146 | 1.561 |

Toxicity to brine shrimp

Toxicity parameters for assays in which shrimp larvae are exposed to the compound in saline solution are shown in Table 3. The obtained values show good correlation (g < 0,4) and high $LC_{50}$ values, indicating a low toxicity for this type of organisms.

Table 3 - Toxicity parameters of compounds tested on brine shrimp larvae, method F.

| Compound No. | $LC_{50}$ (μg/mL) | Confidence Intervals at 95% for $LC_{50}$ (μg/mL) | No. of Organisms Tested | Slope | g |
|---|---|---|---|---|---|
| **Method F** | | | | | |
| **1** | 38.36 | 8.244 - 3258.70 (90%) | 60 | 0.423±0.175 | 0.655 |
| **Imidacloprid** | 0.03121 | 0.02387 - 0.04593 | 100 | 2.435+0.802 | 0.417 |

Insecticidal effect on the adult whitefly

Several compounds were tested, which were applied by spraying 600 μL solutions of each of the compounds (prepared according to Method G) on the leaves of tomato plants infested with a determined number of adult whiteflies *T. vaporariorum.* 14 hours later, the number of dead insects (or, optionally, disappeared) was counted. These assays were carried out at room temperature, between 20 and 25°C.
It was found that the tested compounds were effective as insecticides.

## Discussion and conclusions

Bioassays were performed using a range of compounds and treatment techniques in different species of arthropods. Adult fruit flies, treated topically, showed high levels of sensitivity to the tested compounds, such that the $LD_{50}$ values determined are much lower than that for the reference insecticide "imidacloprid". However some variation was observed in the toxicity effect produced by the different compounds.
The slope of the linear regression was generally small and of lower value than that obtained for "imidacloprid". When the employed test was the fly feeding method, the compounds were shown to be less toxic. Topical treatment of the larvae did also lead to a lower toxicity of the compounds. However, the slope of linear regression increased uniformly.
Some of the compounds were tested by topical application on the adult housefly and were shown to be less toxic compared to the activity found on the adult fruit fly.
Linear regression slopes were similar to those obtained by the same type of treatment used in the adult fruit fly, which suggests a similar mechanism of action although with less activity and some selectivity.
Contrary to the high insecticidal activity found, the compounds show a very low toxicity against brine shrimps.
The high values of $LC_{50}$ are associated with high linear regression slopes.
It can be concluded that these compounds show a very low toxicity towards this type of organisms in saline medium, causing no toxicity in such ecosystems.
In the tests of spraying the compounds on leaves infested with adult whiteflies it was found that the compounds show a promising activity against the whitefly. These compounds also showed high toxicity against adult fruit flies.

## References

1. Haynes, L. J.; Plimmer, J. R Q. Rev. Chem. Soc. 1960, 14, 292-315.

2. Devon, T. K.; Scott, A. I. Handbook of Naturally Occurring Compounds; Academic Press: New York, 1972; Vol. II, pp. 79-175 (quoted in Ref. 8).

3. Marshall, P. G. In Chemistry of Carbon Compounds; Rodd, E. H., Ed.; Elsevier: New York, 1970; Vol. II D, Chapter 17 (quoted in Ref. 8).

4. (a) Schmitz, F. J.; Kraus, K. W.; Ciereszko, L. S.; Sifford, D. H.; Weinheimer, A. J. Tetrahedron Lett. 1966, 7, 97-104; (b) Cimino, G.; De Stefano, S.; Minale, L.; Fattorusso, E. Tetrahedron 1972, 28, 333-341; (c) Cafieri, F.; Fattorusso, E.; Santacroce, C.; Minale, L.; Tetrahedron 1972, 28, 1579-1583; (d) Faulkner, D. J. Tetrahedron Lett. 1973, 14, 3821-3822; (e) Rothberg, I.; Shubiak, P. Tetrahedron Lett. 1975, 16, 769-722; (f) Cimino, G.; De Stefano, S.; Guerriero, A.; Minale, L. Tetrahedron Lett. 1975, 16, 1417-1420.

5. Ma, S.; Schi, Z. ; Yu, Z. Tetrahedron 1999, 55, 12137-12148.

6. Larock, R. C. ; Riefling, B.; Fellows, C. A. J. Org. Chem. 1978, 43, 131-137.

7. Brownbridge, P.; Chan, T. H. Tetrahedron Lett. 1980, 21, 3431-3434.

8. Cardellach, J.; Estopa, C.; Font, J.; Moreno-Mañas, M.; Ortuño, R. M.; Sachez-Ferrando, F.; Valle, S.; Vilamajo, L. Tetrahedron 1982, 38, 2377-2394.

9. Kotora, M.; Negishi, E. Synthesis 1997, 121-128.

10. Klein Gebbinck, E. A.; Stork, G. A.; Jansen, B. J. M.; de Groot, A. Tetrahedron 1999, 55, 11077-11094.

11. Choudhury, P. K.; Foubelo, F.; Yus, M. Tetrahedron 1999, 55, 10779-10788.

12. Figueredo, M.; Font, J.; Virgili, A. Tetrahedron 1987, 43, 1881-1886.

13. Rauter, A. P.; Ferreira, M. J.; Font, J.; Virgili, A.; Figueredo, M.; Figueiredo, J. A.; Ismael, M. I.; Canda, T. L. J. Carbohydr. Chem. 1995, 14, 929-948.

14. Rauter, A. P.; Figueiredo, J. A.; Ismael, M. I.; Pais, M. S.; Gonzalez, A. G.; Dias, J.; Barrera, J. B. J. Carbohydr. Chem. 1987, 6, 259-272.

15. Rauter, A. P.; Figueiredo, J.; Ismael, M.; Canda, T. L.; Font, J.; Figueredo, M. Tetrahedron: Asymmetry 2001, 12, 1131-1146.

16. Csuk, R.; Furstner, A.; Weidmann, H. J. Chem. Soc., Chem. Commun. 1986, 775.

17. Csuk, R.; Glänzer, B. I. ; Hu, Z.; Boese, R. Tetrahedron 1994, 50, 1111-1124.

18. Hanessian, S.; Girard, C. Synlett 1994, 10, 865-867.

19. Rao, A. S. Tetrahedron 1983, 39, 2323-2367.

20. Garem, B. Tetrahedron 1978, 34, 3353-3383.

21. Rodrigues, J.; Dulcere, J. P. Synthesis, 1993, 1177-1202.

22. Waggins, L. F. Nature, 1950, 165.

23. Ohle, M.; Vargha, L. V. J. Chem. Soc. 1959, 2717.

24. Mitsunobu, O. Synthesis 1981, 1.

25. Szeja, W. Synthesis 1985, 983-985.

26. Kwart, H.; Hoffman, D. M. J. Org. Chem. 1966, 31, 419- 425.

27. Gutshe, C. D. Organic Reactions, 1954, 8, 364.

28. Rauter, A. P.; Figueiredo, J. A.; Ismael, M. I., Carbohydr. Res. 1989, 188, 19-24.

29. Dax, K.; Rauter, A. P.; Stütz, A. E.; Weidmann, H., Liebigs Ann. Chem. 1981, 1768-1773.

30. Robertson, J. L.; Preisher, H., Pesticide Bioassays with Arthropods, 1992, CRC Press, Boca Raton, FL.

31. Finney, D. J., Probit Analysis, 1972, 3rd Ed., Cambridge Univesity Press, London, p. 79.

**Claims**

**1.** Compound of the formula (I)

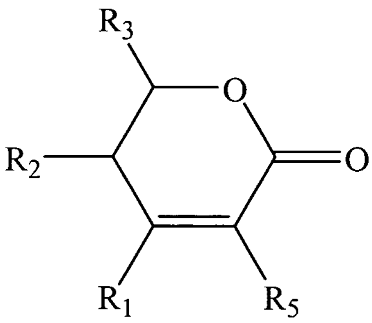

(I)

wherein

$R_1$ and $R_2$ represent, independently from each other, hydrogen, halogen, alkoxy, substituted alkoxy or an ester group; or
$R_3$ represents $-CH_2R_6$,
wherein

$R_6$ represents an ester group,

oxiranyl,
or a group of formula

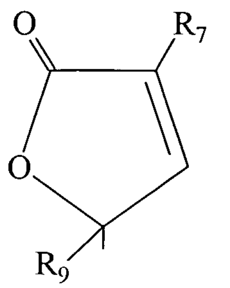

or

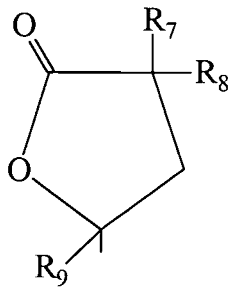

wherein

$R_7$ represents hydrogen or alkyl,
$R_8$ represents phenylsulfanyl, phenylselenyl, phenylsulfoxy or phenylselenoxy, and
$R_9$ represents hydrogen, ethoxycarbonyl or carbamoyl; and

$R_5$ represents hydrogen or halogen.

**2.** Compound according to claim 1, which presents formula (I')

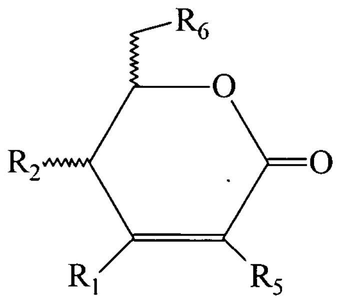

(I')

wherein $R_1$, $R_2$, $R_5$ and $R_6$ have the meanings defined in claim 1.

**3.** Compound according to claim 2 wherein $R_1$ represents hydrogen, $R_2$ represents -OC (=O) $CH_3$, $R_5$ represents Br and $R_6$ represents -OC(=O)$CH_3$.

**4.** Compound according to claim 3 which is a D-*eritro* derivative, *i.e.*, 4,6-di-*O*-acetyl-2-bromo-2,3-dideoxy-D-eritro-hex-2-enono-1,5-lactone.

**5.** Process for the preparation of a compound of any one of claims 2 to 4 which comprises converting a compound of formula (II) into an $\alpha,\beta$-unsaturated hexono-1,5-lactone of formula (I'') [compound of formula (I'), in which $R_1$ represents hydrogen, $R_2$ and $R_6$ represent acetoxy and $R_5$ represents bromine], according to the following scheme:

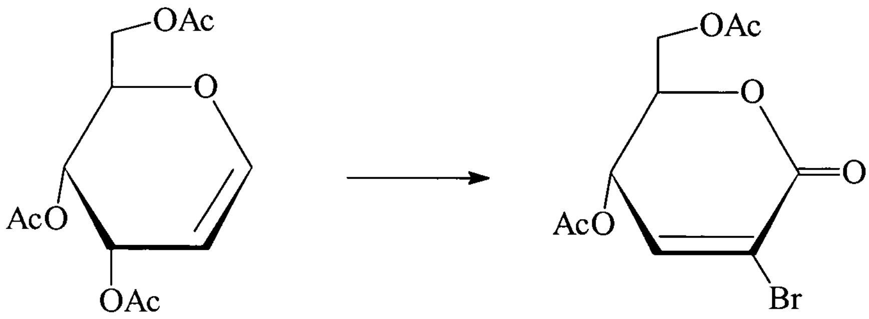

(II) (I'')

**6.** Use of a compound of any one of claims 1 to 4 as a pesticide.

**7.** Use according to claim 6 wherein the pests are arthropods.

**8.** Use according to claim 7 wherein the arthropods are insects.

**9.** Use according to claim 8 wherein the insects are fruit fly *(Drosophila melanogaster),* housefly (*Musca domestica*) and whitefly (*Trialeurodes vaporarium*).

**10.** Method to control pests comprising the application of an effective amount of a compound of any one of claims 1 to 4, to the said pests or their locus.

**11.** Method according to claim 10 wherein the pests are arthropods.

**12.** Method according to claim 11 wherein the arthropods are insects.

**13.** Method according to claim 12 wherein the insects are the fruit fly *(Drosophila melanogaster),* housefly (*Musca*

*domestica*) or whitefly (*Trialeurodes vaporarium*) .

Europäisches Patentamt
European Patent Office
Office européen des brevets

# EUROPEAN SEARCH REPORT

Application Number
EP 09 00 2251

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | RAUTER A P ET AL: "Efficient synthesis of alpha,beta-unsaturated gamma-lactones linked to sugars" TETRAHEDRON: ASYMMETRY, ELSEVIER SCIENCE PUBLISHERS, AMSTERDAM, NL, vol. 12, no. 8, 21 May 2001 (2001-05-21), pages 1131-1146, XP004245861 ISSN: 0957-4166 * the whole document * | 1,6 | INV. C07H7/06 C07H9/04 C07D309/32 C07H3/10 A01N43/08 A01N43/16 A01N43/90 |
| X | PANFIL I ET AL: "1,3-DIPOLAR CYCLOADDITION OF NITRONES TO SUGAR ENLACTONES" TETRAHEDRON, ELSEVIER SCIENCE PUBLISHERS, AMSTERDAM, NL, vol. 47, no. 48, 1991, pages 10087-10094, XP002041211 ISSN: 0040-4020 compounds 2-4 | 1-5 | |
| X | J. LEE ET AL.: "Conformationally constrained analogues of diacylglycerol. 13. Protein kinase C ligands based on templates derived from 2,3-dideoxy-L-erythro(threo)-hexono-1,4-lactone and 2-deoxyapiolactone" J. MED. CHEM., vol. 39, 1996, pages 4912-4919, XP002419334 compound 8 | 1-5 | TECHNICAL FIELDS SEARCHED (IPC) C07H C07D A01N |
| X | R.D. EVANS, J.H. SCHAUBLE: "Synthesis of alfa-iodocarbonyl compounds using bis(sym-collidine)iodine(I) tetrafluoroborate/dimethyl sulfoxide" SYNTHESIS, 1986, pages 727-730, XP002419335 compound 28 | 1-5 | |

The present search report has been drawn up for all claims

1

| Place of search | Date of completion of the search | Examiner |
|---|---|---|
| The Hague | 22 February 2010 | de Nooy, Arjan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Haynes, L. J. ; Plimmer, J. R Q.** *Rev. Chem. Soc.,* 1960, vol. 14, 292-315
- **Devon, T. K. ; Scott, A. I.** Handbook of Naturally Occurring Compounds. Academic Press, 1972, vol. II, 79-175
- **Marshall, P. G.** Chemistry of Carbon Compounds. Elsevier, 1970, vol. II
- **Schmitz, F. J. ; Kraus, K. W. ; Ciereszko, L. S. ; Sifford, D. H. ; Weinheimer, A. J.** *Tetrahedron Lett.,* 1966, vol. 7, 97-104
- **Cimino, G. ; De Stefano, S. ; Minale, L. ; Fattorusso, E.** *Tetrahedron,* 1972, vol. 28, 333-341
- **Cafieri, F. ; Fattorusso, E. ; Santacroce, C. ; Minale, L.** *Tetrahedron,* 1972, vol. 28, 1579-1583
- **Faulkner, D. J.** *Tetrahedron Lett.,* 1973, vol. 14, 3821-3822
- **Rothberg, I. ; Shubiak, P.** *Tetrahedron Lett.,* 1975, vol. 16, 769-722
- **Cimino, G. ; De Stefano, S. ; Guerriero, A. ; Minale, L.** *Tetrahedron Lett.,* 1975, vol. 16, 1417-1420
- **Ma, S. ; Schi, Z. ; Yu, Z.** *Tetrahedron,* 1999, vol. 55, 12137-12148
- **Larock, R. C. ; Riefling, B. ; Fellows, C. A.** *J. Org. Chem.,* 1978, vol. 43, 131-137
- **Brownbridge, P. ; Chan, T. H.** *Tetrahedron Lett.,* 1980, vol. 21, 3431-3434
- **Cardellach, J. ; Estopa, C. ; Font, J. ; Moreno-Mañas, M. ; Ortuño, R. M. ; Sachez-Ferrando, F. ; Valle, S. ; Vilamajo, L.** *Tetrahedron,* 1982, vol. 38, 2377-2394
- **Kotora, M. ; Negishi, E.** *Synthesis,* 1997, 121-128
- **Klein Gebbinck, E. A. ; Stork, G. A. ; Jansen, B. J. M. ; de Groot, A.** *Tetrahedron,* 1999, vol. 55, 11077-11094
- **Choudhury, P. K. ; Foubelo, F. ; Yus, M.** *Tetrahedron,* 1999, vol. 55, 10779-10788
- **Figueredo, M. ; Font, J. ; Virgili, A.** *Tetrahedron,* 1987, vol. 43, 1881-1886
- **Rauter, A. P. ; Ferreira, M. J. ; Font, J. ; Virgili, A. ; Figueredo, M. ; Figueiredo, J. A. ; Ismael, M. I. ; Canda, T. L. J.** *Carbohydr. Chem.,* 1995, vol. 14, 929-948
- **Rauter, A. P. ; Figueiredo, J. A. ; Ismael, M. I. ; Pais, M. S. ; Gonzalez, A. G. ; Dias, J. ; Barrera, J. B.** *J. Carbohydr. Chem.,* 1987, vol. 6, 259-272
- **Rauter, A. P. ; Figueiredo, J. ; Ismael, M. ; Canda, T. L. ; Font, J. ; Figueredo, M.** *Tetrahedron: Asymmetry,* 2001, vol. 12, 1131-1146
- **Csuk, R. ; Furstner, A. ; Weidmann, H.** *J. Chem. Soc., Chem. Commun.,* 1986, 775
- **Csuk, R. ; Glänzer, B. I. ; Hu, Z. ; Boese, R.** *Tetrahedron,* 1994, vol. 50, 1111-1124
- **Hanessian, S. ; Girard, C.** *Synlett,* 1994, vol. 10, 865-867
- **Rao, A. S.** *Tetrahedron,* 1993, vol. 39, 2323-2367
- **Garem, B.** *Tetrahedron,* 1978, vol. 34, 3353-3383
- **Rodrigues, J. ; Dulcere, J. P.** *Synthesis,* 1993, 1177-1202
- **Waggins, L. F.** *Nature,* 1950, 165
- **Ohle, M. ; Vargha, L. V.** *J. Chem. Soc.,* 1959, 2717
- **Mitsunobu, O.** *Synthesis,* 1981, 1
- **Szeja, W.** *Synthesis,* 1985, 983-985
- **Kwart, H. ; Hoffman, D. M.** *J. Org. Chem.,* 1966, vol. 31, 419-425
- **Gutshe, C. D.** *Organic Reactions,* 1954, vol. 8, 364
- **Rauter, A. P. ; Figueiredo, J. A. ; Ismael, M. I.** *Carbohydr. Res.,* 1989, vol. 188, 19-24
- **Dax, K. ; Rauter, A. P. ; Stütz, A. E. ; Weidmann, H.** *Liebigs Ann. Chem.,* 1981, 1768-1773
- **Robertson, J. L. ; Preisher, H.** Pesticide Bioassays with Arthropods. CRC Press, 1992
- **Finney, D. J.** Probit Analysis. Cambridge Univesity Press, 1972, 79